# EUROPÄISCHE PATENTSCHRIFT

(11) **EP 3 651 692 B1**
(45) Veröffentlichungstag und Bekanntmachung des Hinweises auf die Patenterteilung: **03.09.2025**
(21) Anmeldenummer: 18755135.3
(22) Anmeldetag: 13.07.2018
(51) Int. Cl.: A61F 2/07, A61F 2/06

(54) **INTRALUMINALE GEFAESSPROTHESE**
INTRALUMINAL VASCULAR PROSTHESIS
PROTHÈSE VASCULAIRE INTRALUMINALE

(30) Priorität: 14.07.2017 DE 102017115898
(43) Veröffentlichungstag der Anmeldung: 20.05.2020
(73) Patentinhaber: JOTEC GmbH, 72379 Hechingen (DE)
(72) Erfinder: BARTHOLD, Franz-Peter, 72336 Balingen (DE)
(74) Vertreter: Witte, Weller & Partner Patentanwälte mbB
(86) Internationale Anmeldenummer: PCT/EP2018/069123
(87) Internationale Veröffentlichungsnummer: WO 2019/012123

(56) Entgegenhaltungen:
- EP-A2- 3 315 092
- US-A1- 2009 264 977
- US-A1- 2013 282 102
- US-A1- 2014 277 347

## Beschreibung

Die vorliegende Erfindung betrifft eine intraluminale Gefäßprothese zur Implantation in ein Blutgefäß, mit einem Stent-Gerüst und einem an dem Stent-Gerüst befestigten Prothesenmaterial, wobei die Gefäßprothese einen hohlzylindrischen Grundkörper mit einem hierdurch führenden Lumen und einem umfänglich geschlossenem Mantel umfasst.

Allgemein sind intraluminale Gefäßprothesen bekannt, die auch als endovaskuläre Gefäßstents/-stentgrafts bezeichnet werden. Diese werden zur Behandlung von Aneurysmen in Arterien implantiert. Es handelt sich hierbei um Gefäßprothesen die auch allgemein zur Unterstützung von labilen, brüchigen bzw. thrombotischen Gefäßwänden eingesetzt werden. Zur Behandlung wird an der erkrankten bzw. verletzten Stelle des Gefäßes eine Gefäßprothese freigesetzt, die die Funktionalität des ursprünglichen Gefäßes wieder herstellt bzw. die noch bestehende Gefäßintegrität unterstützt. Die US 2014/0277347 A1 offenbart Endoprothesen mit einer fenestrierten Seitenöffnung.

Innerhalb dieser Erfindung, aber auch allgemein, versteht man unter einem Aneurysma eine Ausweitung oder Aussackung eines arteriellen Blutgefäßes infolge angeborener oder erworbener Wandveränderungen. Wandveränderungen treten beispielsweise durch ein sehr rasches Wachstum der Gefäße auf. Beispielsweise wächst die Aorta eines Menschen lebenslänglich, so ist der Durchmesser der Aorta bei 70-Jährigen um 20-30% größer als bei 20-Jährigen. 75% aller Aneurysmen sind in der abdominalen Aorta lokalisiert. Eine Aussackung kann die Gefäßwand als Ganzes erfassen oder, wie bei dem sog. falschen Aneurysma bzw. der sog. Dissektion, es tritt Blut aus dem Lumen des Gefäßes zwischen die Gefäßwandschichten und schert diese auseinander. Die Nichtbehandlung eines Aneurysma kann im fortgeschrittenen Stadium zu einer Ruptur der Arterie führen, mit der Folge, dass der Patient innerlich verblutet.

Weitere Ursachen eines thorakalen und thorakoabdominalen Aortenaneurysmas können Arteriosklerose, Bluthochdruck sowie Entzündungsprozesse der Gefäßwand sein. Auch Verletzungen des Brustkorbes durch schwere Unfälle können akut oder chronisch zu einem Aortenaneurysma führen.

Zur Behandlung von Aneurysmen werden, gemäß dem Stand der Technik, die betroffenen Arterien durch Implantation eines Stents/Stentgrafts stabilisiert, um eine Ruptur des Gefäßes zu vermeiden. Je nach Anwendungsart werden unterschiedliche Gefäßprothesen eingesetzt. Im Allgemeinen unterscheidet man zwischen ballonexpandierbaren und selbstexpandierbaren System sowie solchen ohne und mit Prothesenmaterial. Bei den Letztgenannten spricht man auch von "gecoverten" Stents. Das Prothesenmaterial ist häufig als Textil- bzw. als Polymerfolie ausgestaltet und verhindert insbesondere ein Blutdurchtritt bzw. ein Durchtritt von Blutbestandteilen oder Ablagerungen durch die Wandung der Gefäßprothese, sowie das Einwachsen von Gewebe durch die Wandung in das Innere der Gefäßprothese. Durch diese Ausgestaltung wird die Gefäßwand an der Implantationsstelle des Stentgrafts entlastet und mögliche Embolien an diesen Stellen können verhindert werden.

Der röhrchenförmigen Metallrahmen, dessen Mantelfläche mit einer Textil- oder Polymerfolie größtenteils abgedeckt sein kann, ist derart geformt, dass sich ein hohlzylindrischer Körper ergibt. Der Metallrahmen besteht meist aus einem Drahtgeflecht oder aus hintereinander angeordneten, mäanderförmig umlaufenden Stent-Elemente, die auch als Stent-Federn bekannt sind, die ggf. über Verbindungsstützen miteinander verbunden sind, oder die lediglich über das Prothesenmaterial miteinander verbunden sind. Sofern es sich um eine selbstexpandierbares Gefäßprothese handelt, ist das Drahtgeflecht bzw. die Stent-Elemente aus einem Shape-Memory-Material bzw. aus einer Formgedächtnislegierung; bspw. aus Nitinol.

Zur Implantation wird die Gefäßprothese radial zusammengedrückt, so dass sich ihre Querschnittsfläche deutlich verringert. Hierfür wird die Gefäßprothese zuvor in eine Hülle dem so genannten Hüllkatheter eingebracht. Der Hüllkatheter ist Teil des Einführsystems, mit dem die Gefäßprothese in den Bereich des Aneurysmas gebracht wird, wo die Gefäßprothese freigesetzt wird. Die Position der Gefäßprothese wird in der Regel über Röntgenmarker bestimmt, sodass die Gefäßprothese ggf. justiert werden kann. Aufgrund der Federwirkung des Metallrahmens expandiert die Gefäßprothese wieder in ihre ursprüngliche Form und spannt dabei ihre Mantelfläche auf, die sich proximal und distal von dem Aneurysma innen in dem Blutgefäß verklemmt. Auf diese Weise fließt das Blut jetzt durch die Gefäßprothese, wodurch eine weitere Belastung der Aussackung verhindert wird. Die Gefäßprothese ist dabei aufgrund ihrer nach außen wirkenden Druckkraft lagefest in der gewünschten Position im Gefäß positioniert. Die Expansion des Metallrahmens kann einerseits durch die Verwendung von selbstexpandierendem Metall, wie bspw. Nitinol, bewirkt werden, oder bei ballonexpandierbaren Gefäßprothesen durch den Einsatz eines Dilatations-Ballons, der von innen in den Metallrahmen eingeführt wird und durch dessen Dilatation der Metallrahmen expandiert wird.

Problematisch ist häufig, dass das zu behandelnde Blutgefäß weitere seitlich abgehende Blutgefäße aufweist. Sofern eine Gefäßprothese in ein solches Gefäß implantiert wird, würden die abgehenden Seitenblutgefäße durch das blutdichte Prothesenmaterial von der Blutversorgung abgeschnitten. Gelöst wird dieses Problem durch sog. "Fenestrierungen" die sich auf dem Prothesenmaterial befinden. Unter fenestrierten Gefäßprothesen werden solche verstanden, die präformierte Löcher (Fenestrationen) aufweisen, um ein oder mehrere Gefäßabgänge in der Gefäßprothese zu ermöglichen.

Alternativ sind solche Gefäßprothesen bekannt, bei denen die Fenestrierung erst insitu eingebracht wird, also nachdem die Gefäßprothesen im Gefäß positioniert wurden. Eine derartige Gefäßprothese ist bspw. aus der WO 2009/064672 A2 bekannt. Hier wird der Haupt-Stentgraft in situ mit einer Nadel penetriert, um ein Nadelloch im Graft-Material zu bilden. Anschließend wird eine Dilator-Anordnung durch das Nadelloch geschoben, um das Nadelloch zu erweitern. Nachteilig bei diesen Gefäßprothesen ist insbesondere, dass das Prothesenmaterial an vorgesehenen Positionen zerrissen bzw. verletzt wird, um die Fenestrierungen zu bilden. Dadurch besteht die Gefahr des weiteren Ausreißens des Prothesenmaterials, so dass sich die Fenestrierung unkontrolliert erweitert, wodurch es als Folge wiederum zu einem unkontrollierten Ausfließen des Blutes in diesem Bereich der Gefäßprothese kommen kann. Ein weiterer Nachteil bei den im Stand der Technik bekannten Prothesen und Verfahren ist es, dass die Position des abgehenden Gefäßes nicht mit Kontrastmitteln zu lokalisieren ist.

Nachteile der im Stand der Technik bekannten Gefäßprothesen bestehen darin, dass diese sehr korrekt in Bezug auf die abzweigenden Gefäße positioniert werden müssen, da sich ansonsten eine Aussackung im Bereich der Löcher ausbilden kann, während die Seitengefäße von der Blutversorgung abgeschlossen werden. Dies erfordert viel Erfahrung von dem behandelnden Arzt.

Insbesondere bei solchen Gefäßprothesen, die mehrere abgehende weitere Seitengefäße überbrücken soll, ist es meist notwendig, ein auf den jeweiligen Patienten bzw. dessen Gefäß genau zugeschnittene Gefäßprothesen bereitzustellen, wenn eine erfolgreiche Behandlung erreicht werden soll. Dies ist besonders kostenintensiv und zeitaufwendig, da das zu behandelnde Gefäß zunächst in seiner genauen Beschaffenheit hin untersucht werden muss.

Im Stand der Technik sind ferner Gefäßprothesen bekannt, die im Prothesenmaterial gitterartige Fenestrierungen aufweisen, über welche Seitenäste eingeführt werden können. Über diese "mini-Fenestrierungen" werden temporäre Endoleaks geschaffen, da über diese "Löcher" im Gewebe das Blut für eine bestimmte Zeit auslaufen kann, um die Sondierung des abgehenden Gewebes überhaupt erst möglich zu machen. Bei diesen Modellen sind viele kleine "Löcher" bzw. Fenestrierungsstellen über einen Abschnitt der Prothese verteilt. Nach erfolgter in-situ Fenestrierung sollen sich die restlichen, dann nicht verwendeten Löcher durch Blutgerinnung verschließen und die Endoleckage beenden.

Diese Endoleckagen stellen aber bereits *per se* einen Nachteil dar, da über diese unkontrolliert Blut aus der Prothese austritt. Ferner dürfen diese gitter-/siebartigen Fenestrierungslöcher nicht zu groß gestaltet sein, da ansonsten wiederum die Endoleckage zu groß ist. Dies birgt allerdings den Nachteil, dass bei der Legung von größeren Seitenästen die Gefahr des Einreißens des Materials besteht.

Aufgabe der vorliegenden Erfindung ist es daher, eine intraluminale Gefäßprothese bzw. einen Stentgraft bereitzustellen, mit dem die oben geschilderten Nachteile überwunden werden können, und mit welchem Gefäßprothesen bereitgestellt werden können, die flexibel, d.h. nicht maßgeschneidert angefertigt werden müssen, wobei gleichzeitig die Dimensionen der Fenestrierungen gewährleistet bleiben.

Erfindungsgemäß wird diese Aufgabe durch eine intraluminale Gefäßprothese zur Implantation in ein Blutgefäß, mit einem Stent-Gerüst und einem an dem Stent-Gerüst befestigten Prothesenmaterial gelöst, wobei die Gefäßprothese einen hohlzylindrischen Grundkörper mit einem hierdurch führenden Lumen und einem umfänglich geschlossenem Mantel umfasst, dadurch gekennzeichnet, dass im Prothesenmaterial der Gefäßprothese zumindest ein im Wesentlichen U- oder V-förmiger Fenestrierungs-Schnitt vorgesehen ist, der derart dimensioniert und ausgebildet ist, dass über diesen zum Lumen der Gefäßprothese ein klappenartigen Zugang für zumindest einen von dem hohlzylindrische Grundkörper abgehenden Seitenast ausbildbar ist, und dass das Stent-Gerüst der intraluminalen Gefäßprothese aus in ihrer Längsrichtung hintereinander angeordneten, aber nicht miteinander verbundenen Ringen aus mäanderförmig umlaufenden Stützen besteht und der zumindest eine Fenestrierungs-Schnitt in einem Umfangsabschnitt U zwischen zwei hintereinander angeordneten Ringen vorgesehen ist, und dass am ersten und am zweiten Ende der Gefäßprothese jeweils ein Stentring vorgesehen ist, der mit dem Stent-Gerüst verbunden ist.

Die der Erfindung zugrundeliegende Aufgabe wird dadurch vollkommen gelöst.

Mit der erfindungsgemäßen intraluminalen Gefäßprothese wird eine Gefäßprothese bereitgestellt, welche zur Unterstützung von labilen, brüchigen bzw. thrombischen Gefäßwänden und insbesondere zur Behandlung von aneurysmatischen Gefäßen eingesetzt werden kann. Dies wird erreicht durch den speziellen Aufbau der erfindungsgemäßen Gefäßprothese, insbesondere durch die klappenartigen Zugang erreicht: Die Klappen öffnen sich nur an den Stellen nach außen, an denen tatsächlich auch ein abgehendes Gefäß in der Nähe ist bzw. unmittelbar daran anschließt. Die anderen Klappen bleiben durch den Wandkontakt verschlossen und verringern so die Gefahr von unerwünschten Endoleaks.

Ein weitere Vorteil besteht darin, dass die Öffnung der Klappen wesentlich größer gewählt werden kann als dies der Fall bei gitter-/siebartigen Fenestrierungs-Löchern, wie weiter oben beschrieben, der Fall ist. Dies wiederum verringert die Gefahr des Ausreißens des Prothesenmaterials.

Dabei bedeutet hier - wie auch überhaupt vorliegend - "im Wesentlichen", dass der U- oder V-förmiger Fenestrierungs-Schnitt nicht exakt die Form des Buchstabens U oder V aufweist, sondern dass auch Formen damit umfasst sind, die ein Fachmann als annähernd U- oder V-förmig erkennen und einordnen wird.

Der Fenestrierungs-Schnitt und damit der klappenartigen Zugang ermöglicht es, dass die Blutversorgung der seitlich abgehenden Seitengefäße gewährleistet werden kann. Die Gefäßprothese wird hierfür derart in das Gefäß gelegt, dass zumindest ein klappenartiger Zugang dem abgehenden Seitenast zugewandt ist. Besonders vorteilhaft hierbei ist, dass die Blutversorgung zum einen erreicht werden kann, durch bloßes Aufklappen des Zugangs. Hierbei wird das Prothesenmaterial, welches sich innerhalb des U- bzw. V-förmigen Fenestrierungs-Schnitt befindet an die Gefäßwand des abgehenden Seitenastes aufgrund des Blutstroms gedrückt. Zum anderen kann durch den klappenartigen Zugang eine weitere Gefäßprothese gelegt werden. Auch hierdurch wird die Versorgung der Seitengefäße gewährleistet.

Darüberhinaus bietet die erfindungsgemäße Gefäßprothese den Vorteil, dass die Gefäßprothese deutliche mehr Fenestrierungs-Schnitte aufweisen kann, als das Gefäß abgehende Seitengefäße aufweist. Im implantierten Zustand liegen hierbei die klappenartigen Zugänge an der Gefäßwand an, wobei kein Blut durch die Öffnung nach außen fließt. Die Klappe lässt sich demnach selbst öffnen, sofern sich ein Seitenarm eines Seitengefäßes hinter der Klappe befindet. Die Klappe lässt sich demnach an den gewünschten Stellen im Gefäß leicht öffnen und bleibt geschlossen an den Stellen, an denen sich kein Seitenast befindet. Durch diesen speziellen Aufbau wird auch ein gezieltes Verklotten ermöglicht.

Ferner wird durch den speziellen erfindungsgemäßen Aufbau der Gefäßprothese ein Auftreten von Endoleaks minimiert. Ein Endoleak oder auch Endoleckage genannt, entspricht einem Leck zwischen einer eingesetzten Prothese und dem Aneurysmasack. Das Endoleak ist mit 15% die häufigste Komplikation nach endovaskulärer Behandlung eines Aortenaneurysmas. Der Blutfluss im Aneurysmasack persistiert, weil das Aneurysma nur inkomplett ausgeschaltet ist. Es besteht weiterhin das Risiko einer Expansion und das Risiko der Ruptur.

Demnach hat die erfindungsgemäße Gefäßprothese den Vorteil, dass diese zum einen für jeden Patienten passgenau gefertigt werden kann, also entsprechend so viele Fenestrierungs-Schnitte aufweist, wie das Gefäß abgehende Seitengefäße aufweist, sondern anderseits dass die Gefäßprothese auch standardisiert hergestellt werden kann, sodass diese universell einsetzbar ist, mit einer Vielzahl an Fenestrierungs-Schnitten.

Das Stent-Gerüst dient zum einen zum befestigen des Prothesenmaterials, zum anderen gibt das Stent-Gerüst der Gefäßprothese die hohlzylindrische Struktur. Außerdem hält das Stent-Gerüst die Gefäßprothese im Gefäß in Position, indem es im implantierten Zustand die Gefäßprothese an die Gefäßwand drückt.

Vorliegend ist mit hohlzylindrischem Grundkörper der Hauptkörper der Gefäßprothese gemeint, welcher aus einem Stent-Gerüst und zumindest teilweise aus Prothesenmaterial besteht. Das Stent-Gerüst kann wiederum aus einzelnen Stent-Elementen bestehen.

Gemäß einer Ausführungsform ist bevorzugt, wenn die intraluminale Gefäßprothese auf einem Umfangsabschnitt U insgesamt zwischen einem und neun Fenestrierungs-Schnitten aufweist.

Demnach können erfindungsgemäß 1, 2, 3, 4, 5, 6, 7, 8 oder 9 Fenestrierungs-Schnitte vorgesehen, die zumindest über einen Umfangsabschnitt U der Gefäßprothese verteilt sind. Durch eine größere Anzahl von Fenestrierungs-Schnitten über einen bestimmten Umfangsabschnitt der erfindungsgemäßen Gefäßprothese wird der Vorteil geschaffen, dass die genaue Platzierung der Gefäßprothese in Bezug auf ihren genauen Umfang im Gefäß nicht kritisch ist, da über eine höhere Anzahl von Klappen die Wahrscheinlichkeit erhöht ist, dass eine der genau oder zumindest fast genau Klappen über dem abgehenden Gefäß platziert wird. Durch die besondere klappenartige Ausgestaltung der Fenestrierungs-Schnitte bleibt ein Zugang verschlossen, sofern sich das Prothesenmaterial des Fenestrierungs-Schnittes direkt an der Gefäßwand befindet. Je mehr Fenestrierungs-Schnitte eine Gefäßprothese aufweist, desto einfacher kann sich die Implantation und ggf. die Legung der Seitenäste gestalten.

Je nach Beschaffenheit des Gefäßes, in welches die intraluminale Gefäßprothese eingesetzt werden soll, kann es notwendig sein, dass die Gefäßprothese mehrere Umfangsabschnitte aufweist, die wiederum jeweils zwischen 1, 2, 3, 4, 5, 6, 7, 8 oder 9 Fenestrierungs-Schnitte aufweisen.

Die Fenestrierungs-Schnitte können dabei in unterschiedlichen Größen vorliegen. Die Länge des Fenestrierungs-Schnittes bzw. der hierdurch gebildete Durchmesser des klappenartigen Zugangs wird bevorzugt an den Durchmesser der seitlich abgehenden Seitenäste angepasst.

Unter einem "Umfangsabschnitt" U der erfindungsgemäßen Gefäßprothese wird dabei ein umlaufender Flächenabschnitt der Gefäßprothese verstanden, mithin also ein zylindrischer Abschnitt, über welchen der zumindest eine Fenestrierungs-Schnitt verteilt ist.

Erfindungsgemäß ist bei den Gefäßprothesen, die hintereinander angeordnete mäanderförmig umlaufende Stentringe umfassen, die nicht miteinander verbunden sind, und die ein Prothesenmaterial umfassen, an welches die Stentringe befestigt sind, ist der Umfangsbereich durch den Prothesenbereich definiert, der zwischen zwei hintereinander angeordneten Stentringen gebildet wird.

Gemäß einer anderen Ausführungsform umfasst ein Umfangsabschnitt dabei vorzugsweise zwischen 10 mm und 40 mm, vorzugweise zwischen 10 und 20 mm.

Erfindungsgemäß und gemäß einer weiteren Ausführungsform ist bei der Gefäßprothese vorgesehen, dass zwischen 1, 2, 3, 4, 5, 6, 7, 8 oder 9 Fenestrierungs-Schnitte in zwischen 1, 2, 3, 4, 5, 6, 7, 8, 9 oder 10 Umfangsabschnitten vorgesehen sind.

Dabei können gemäß einer Ausführungsform mit mehreren, also zumindest zwei, Umfangsabschnitten, diese entweder eine unterschiedliche Anzahl an Fenestrierungs-Schnitten aufweisen oder eine gleiche Anzahl an Fenestrierungs-Schnitten. Auch können die Fenestrierungs-Schnitte eines ersten Umfangsabschnitts gleich oder unterschiedlich lang zu den Fenestrierungs-Schnitten eines zweiten Umfangsabschnitts sein, sowie auch innerhalb eines ersten und/oder zweiten Umfangsabschnitts gleich oder unterschiedlich lang. Es versteht sich dabei ferner, dass ein Umfangsabschnitt mehrere, also zumindest zwei gleich lange Fenestrierungs-Schnitte aufweisen kann, sowie auch zumindest einen weiteren hierzu unterschiedlichen Fenestrierungs-Schnitt.

Erfindungsgemäß besteht das Stent-Gerüst der intraluminalen Gefäßprothese aus in ihrer Längsrichtung hintereinander angeordneten, aber nicht miteinander verbundenen Ringen aus mäanderförmig umlaufenden Stützen.

Unter "mäanderförmig" wird vorliegend jeder schlingen- bzw. schleifenförmige Verlauf eines Stent-Rings verstanden. Vorliegend wird unter "Stent" bzw. "Stent-Element" oder "Stent-Ring" jede Struktur bezeichnet, die einer Gefäßprothese eine Expansionskraft und/oder eine stützende Funktion verleiht. Entsprechend ist ein Stent-Element daher jedes Element, das die Eigenschaften eines Stent aufweist.

Vorliegend wird unter einer "Stentfeder" jedes einstückige, ringförmige Element verstanden, dass sich aufgrund seines Materials komprimieren lässt und sich nach Entfernung des Kompressionsdrucks wieder federartig expandieren kann. Die Stentfedern weisen dabei einen wellenartigen Umlauf auf, wobei Wellenberg und Wellental eine Phase bilden und sich gegenseitig abwechseln.

Die Stent-Elemente bzw. Stentfedern können gleiche oder unterschiedliche umlaufende Amplituden aufweisen, welche sich aus den gleich langen bzw. unterschiedlich langen Schenkeln der Stentfedern ergeben. Unterschiedlich lange Amplituden, bieten den Vorteil, dass der Stentgraft an die jeweiligen Gefäße und die jeweiligen Beschaffenheit (Krümmungen, abgehende Gefäße, Verjüngungen, etc.) angepasst werden kann.

Die Stent-Elemente können erfindungsgemäß anstelle der einzelnen Stentfedern auch geflochtene, gezwirbelte oder Laser-geschnittene Stent-Elemente umfassen.

In dieser Ausgestaltung sind die einzelnen mäanderförmig umlaufenden Stützen bevorzugt über das Prothesenmaterial verbunden. Hierfür können die mäanderförmig umlaufenden Stützen durch eine Naht am Prothesenmaterial befestigt sein. Die Stützen können dabei so am Prothesenmaterial angeordnet sein, dass sich ein ungestenter Bereich auf dem Prothesenmaterial ergibt.

Bevorzugt wird als Nahtmaterial chirurgischer Faden verwendet. Dieser ist bevorzugt aus Polyester, Polyurethan, Polystyrol, Polytetrafluorethylen ultrahochmolekulargewichtiges Polyethylen (UHMPE), oder Mischungen davon ausgestaltet.

Gemäß einer bevorzugten Ausführungsform erstreckt sich das Stent-Gerüst über die gesamte Länge der Gefäßprothese.

Je nach Gefäß kann es erforderlich sein, dass die Gefäßprothese ein über die gesamte Länge erstreckendes Stent-Gerüst aufweist. Dies kann beispielsweise erreicht werden mittels eines Drahtgeflechts, wobei dieses zumindest teilweise Prothesenmaterial aufweist. In dem Bereich, in dem sich das Prothesenmaterial befindet, können sich die Fenestrierungs-Schnitte innerhalb der sich ausbildenden Maschen vorliegen.

Alternativ zum Drahtgeflecht kann die Gefäßprothese auch mehrere Stent-Elemente aufweisen, die untereinander über Verbindungsstücke miteinander verbunden sind. Auch hierdurch kann ein durchgängiges Stent-Gerüst ausgestaltet werden.

Gemäß einer weiteren Ausführungsform erstreckt sich das Stent-Gerüst nicht über die gesamte Länge der Gefäßprothese, derart, dass zumindest ein ungestenteter Bereich ausgebildet ist.

Diese Ausführungsform bietet den Vorteil, dass je nach Gefäß, in der die Gefäßprothese implantiert werden soll, eine Gefäßprothese passgenau angefertigt werden kann. Variierbar ist hierbei die Anzahl und die Form des Stent-Gerüst bzw. der einzelnen Stent-Elemente, die Menge an Prothesenmaterial, der Durchmesser der Gefäßprothese, das verwendete Material für das Stent-Gerüst bzw. das Prothesenmaterial, die Anzahl der Fenestrierungs-Schnitte etc.

Erfindungsgemäß ist am ersten und am zweiten Ende der Gefäßprothese jeweils ein Stentring vorgesehen, der mit dem Stent-Gerüst verbunden ist.

Das erste bzw. das zweite Ende bezieht sich erfindungsgemäß auf das proximale bzw. distale Ende der Gefäßprothese. Grundsätzlich werden bei Gefäßprothesen zur Bezeichnung der jeweiligen Enden die Begriffe "distal" und "proximal" verwendet, wobei der Begriff "distal" der Teil, bzw. das Ende bezeichnet wird, der/das in Bezug auf den Blutstrom weiter stromabwärts liegt. Der Begriff "proximal" hingegen bezeichnet, wieder in Bezug auf den Blutstrom, einen Teil bzw. das Ende, der/das in Bezug auf den Blutstrom weiter stromaufwärts liegt. Anders ausgedrückt bedeutet der Begriff "distal" in Richtung des Blutstroms, und der Begriff "proximal" der Richtung des Blutstroms entgegengesetzt. Bei Kathetern hingegen, bzw. Einführsystemen bezeichnet der Begriff "distal" das Ende des Katheters bzw. Einführsystems, das in den Patienten eingeführt wird, bzw. das vom Anwender ausgesehen am Entferntesten liegt, und der Begriff "proximal" das Ende, das sich dem Anwender näher zugewandt ist.

Gemäß einer weiteren Ausführungsform weist der zumindest eine Fenestrierungs-Schnitt eine Schnittlänge von zwischen 2 mm bis 10 mm vorzugsweise zwischen 5 mm und 7 mm auf.

Diese Ausführungsform hat den Vorteil, dass der durch den Fenestrierungs-Schnitt gebildete Zugang den üblichen Maß der Seitengefäße entspricht.

Gemäß einer weiteren Ausführungsform weisen, bei Vorliegen von mehreren Fenestrierungs-Schnitten, diese eine gleiche oder unterschiedliche Schnittlänge auf.

Aufgrund der unterschiedlichen Beschaffenheit der Seitengefäße sind auch unterschiedlich große Fenestrierungs-Schnitte notwendig. Ein zu kleiner Schnitt könnte dafür sorgen, dass die Blutversorgung zwar gegeben ist, aber durch einen zu kleinen Zugang könnte der Blutdruck ungünstig beeinflusst werden. Mit dieser Ausgestaltung können sowohl große wie kleine Seitengefäße überbrückt werden.

Gemäß einer weiteren Ausführungsform umfasst die intraluminale Gefäßprothese neben dem hohlzylindrischen Grundköper ferner zumindest einen hohlzylindrischen Seitenkörper, welcher über den klappenartigen Zugang mit der Gefäßprothese verbindbar ist.

Mit "zumindest einem hohlzylindrischen Seitenkörper" ist/sind dabei vorliegend vorzugsweise ein, zwei, drei oder vier Seitenkörper gemeint. Mit dem Seitenkörper weist die Gefäßprothese eine Öffnung auf, wodurch die Versorgung der abgehenden Seitengefäße über die Gefäßprothesen-Seitenkörper sicher gewährleistet werden kann. Diese Ausgestaltung ist besonders vorteilhaft bei Gefäßen, bei denen sich eine Verletzung bzw. Ruptur in der Nähe eines Seitenastes befindet. Hierbei ist es von Vorteil, wenn nicht nur das Hauptgefäß, sondern auch die Seitengefäße mittels einer ersten und zweiten Gefäßprothese unterstütz werden.

Bei der erfindungsgemäßen Gefäßprothese versteht sich allgemein, dass der zumindest eine Seitenast, der über den klappenartigen Zugang zum Lumen der Gefäßprothese in Verbindung mit diesem gebracht wird, sowohl nach außen in Bezug auf die Gefäßprothese als auch nach innen ins Lumen der Gefäßprothese abgehen kann.

Bei dem hohlzylindrischen Seitenkörper kann es sich erfindungsgemäß um eine zweite Gefäßprothese handeln. Hierbei kann diese zweite Gefäßprothese die gleichen Eigenschaften aufweisen wie die bereits beschrieben Gefäßprothese. Es kann sich demnach um eine selbstexpandierbare oder ballonexpandierbare Gefäßprothese handeln, wobei diese optional Prothesenmaterial aufweist. In einer Ausführungsform weist der hohlzylindrische Seitenköper kein Prothesenmaterial auf.

Diese Ausführungsform bietet daher den Vorteil, dass die erfindungsgemäße Gefäßprothese den jeweiligen anatomischen Verhältnissen des zu behandelnden Patienten angeglichen werden kann.

Gemäß einer weiteren Ausführungsform befindet sich auf der intraluminalen Gefäßprothese ein Marker, der ein röntgendichtes Material enthält oder vollständig aus röntgendichtem Material besteht, wobei der Marker insbesondere an den Endpunkten des zumindest einen Fenestrierungs-Schnitts und/oder entlang des Fenestrierungs-Schnitts vorliegen.

Mit Hilfe der Marker, die sich auf spezifischen Stellen der Gefäßprothese befinden, ist es möglich besonders schnell die Lage der Gefäßprothese während und nach der Implantation genau zu bestimmen. Insbesondere sind Marker rund um den Fenestrierungs-Schnitt sinnvoll, da die Gefäßprothese insbesondere in diesem Bereich richtig positioniert werden soll.

Vorzugsweise sind die röntgendichten Marker aus einem oder mehreren der folgenden Materialien, bspw. Gold, Palladium, Tantal, Chrom, Silber, etc.; die Form der Marker kann dabei beliebig sein, bspw. rund eckig, und/oder bspw. die Form von Buchstaben, Zahlen oder Figuren haben, die für die Orientierung des Stentgrafts im Gefäß hilfreich sind.

Die erfindungsgemäße intraluminalen Gefäßprothese kann zur Implantation in ein Blutgefäß eines Patienten, zur Behandlung einer Gefäßerkrankung eingesetzt werden.

Vorliegend offenbart, aber nicht beansprucht ist ein Verfahren zur Einbringung einer intraluminalen Gefäßprothese in ein Blutgefäß eines Patienten, mit den folgenden Schritten:
- Einbringen und Freisetzen einer ersten intraluminalen Gefäßprothese in ein Blutgefäß eines Patienten; und
- Einbringen von zumindest einer zweiten Gefäßprothese über den durch den zumindest einen Fenestrierungs-Schnitt ausgebildeten, klappenartigen Zugang in dem hohlzylindrischen Grundkörper zur Ausbildung von zumindest einem Seitenast der intraluminalen Gefäßprothese in einem von dem Blutgefäß abgehenden Seitengefäß.

Vorliegend offenbart, aber nicht beansprucht ist ein Verfahren zur Erweiterung einer intraluminalen Gefäßprothese, mit den folgenden Schritten:
- Bereitstellung einer intraluminalen Gefäßprothese; und
- Durchdringen durch den zumindest einen Fenestrierungs-Schnitt ausgebildeten, klappenartigen Zugang in dem hohlzylindrischen Grundkörper der intraluminalen Gefäßprothese mit einer zweiten Gefäßprothese zur Ausbildung einer Seitenast-Gefäßprothese.

Außerdem wird die Aufgabe durch ein Verfahren zur Herstellung einer intraluminalen Gefäßprothese gelöst, wobei der Fenestrierungs-Schnitt im Prothesenmaterial mittels einer thermischen Behandlung des Prothesenmaterials erfolgt.

Gemäß einer bevorzugten Ausführungsform des Verfahrens wird der Fenestrierungs-Schnitt mittels eines Laserwerkzeugs oder eines Werkzeugs eingebracht.

Diese Ausführungsform bietet den Vorteil, dass mittels eines Laserwerkzeugs besonders präzise Schnitte durchgeführt werden können. Ferner wird durch die Erwärmung während des Schnittes die Schnittkanten des Prothesenmaterials angeschmolzen, sodass das Gewebe an dieser Stelle nicht unerwünscht ausfranst oder derart scharfe Kanten aufweist, dass möglicherweise die Gefäßwand verletzt werden kann.

Ausführungsbeispiele der Erfindung sind in der Zeichnung dargestellt und werden in der nachfolgenden Beschreibung näher erläutert. Es zeigen:
- Fig. 1: eine erste schematische Darstellung eines Ausschnitts einer erfindungsgemäßen intraluminalen Gefäßprothese;
- Fig. 2: eine zweite schematische Darstellung einer erfindungsgemäßen intraluminalen Gefäßprothese;
- Fig. 3: eine schematische Darstellung eines Ausschnitts einer erfindungsgemäßen intraluminalen Gefäßprothese mit eingeführtem hohlzylindrischem Seitenkörper; und
- Fig. 4: eine beispielhafte, nicht erfindungsgemäße schematische Darstellung eines Ausschnitts einer intraluminalen Gefäßprothese, .

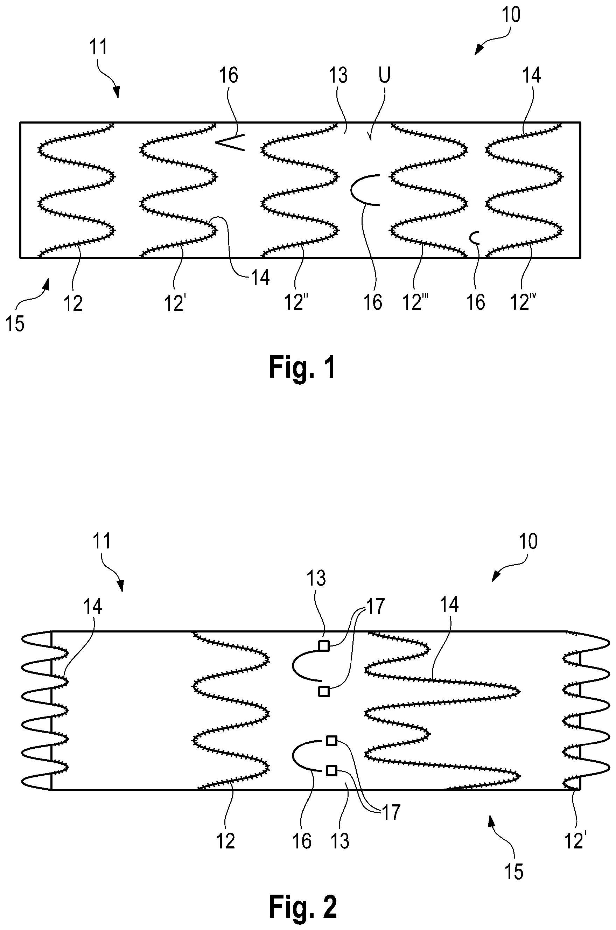

In Fig. 1 ist eine erste schematische Darstellung einer Ausführungsform, eines Ausschnitts einer erfindungsgemäßen intraluminalen Gefäßprothese 10 gezeigt. Diese weist ein Stent-Gerüst 11 auf, welches wiederrum aus einzelnen Stentfedern bzw. StentRingen 12 aufgebaut ist, die aus Ringen aus mäanderförmig umlaufenden Stützen bestehen. Die Stentfedern/Stentringe 12, 12^{I}, 12^{II}, 12^{III}, 12^{IV} in dieser Figur fünf an der Zahl, sind am Prothesenmaterial 13 mittels einer Naht 14 befestigt. Das Prothesenmaterial 13 erstreckt sich in dieser Darstellung über die gesamte Gefäßprothese 10. Die Stent-federn 12 liegen in dieser Ausgestaltung in Längsrichtung hintereinander angeordnet vor und sind untereinander nicht miteinander verbunden, jedoch über das Prothesenmaterial 13. Die Stentfedern 12 sind bevorzugt aus einem Material hergestellt oder weisen ein solches Material auf, welches selbstexpandierbar ist. Die Stentfedern 12 können somit sich von einem komprimierten Zustand von selbst in einen relaxierten, expandierten Zustand bringen. Dies ist bevorzugt um die intraluminale Gefäßprothese 10 in ein Gefäß implantieren zu können.

Das Prothesenmaterial 13 ist als hohlzylindrischer Grundkörper 15 mit einem hierdurch führenden Lumen ausgestaltet, wobei das Prothesenmaterial 13 einen umfänglich geschlossenen Mantel bildet. Die hohlzylindrische Struktur des Grundkörpers 15 wird primär durch das Stent-Gerüst 11 gebildet. Das Stent-Gerüst 11 kann sich bevorzugt über die gesamte Länge der Gefäßprothese 10 erstrecken, da dieses die Festigkeit der Struktur der Gefäßprothese 10 bestimmt. Alternativ kann sich das Stent-Gerüst 11 auch nicht über die gesamte Länge der Gefäßprothese 10 erstrecken. Hierbei wird das Stent-Gerüst 11 durch Prothesenmaterial 13 unterbrochen. In dieser Ausgestaltung wird das Prothesenmaterial 13 bevorzugt an seinen Enden von einem Stent-Gerüst 11 eingerahmt, um der Gefäßprothese 10 eine hohlzylindrische Struktur zu geben.

Das Prothesenmaterial 13 weist in dieser Darstellung drei Fenestrierungs-Schnitte 16 auf. Diese sind U- bzw. V förmig ausgestaltet und weisen eine unterschiedliche Schnittlänge auf. Die Schnittlänge richtet sich bevorzugt nach dem Durchmesser der abgehenden Seitenäste des Hauptgefäßes. Sofern die Gefäßprothese 10 eine zweite Gefäßprothese aufweist, die in den klappenartigen Zugang gelegt werden kann, zur Ausbildung einer Seitenarm-Gefäßprothese, richtet sich die Schnittlänge an den entsprechenden Durchmesser der zweiten Gefäßprothese. Sofern die zweite Gefäßprothese einen kleinen Durchmesser aufweist, ist die Schnittlänge des Fenestrierungs-Schnitts 16 entsprechend kurz.

Ferner ist in Fig. 1 mit "U" ein beispielhafter Umfangsabschnitt bezeichnet, der durch die beiden hintereinander angeordneten, mäanderförmig umlaufenden Stentfedern/Stentringen 12^{II} und 12^{III} gebildet wird. Entsprechend ist die eine Begrenzung des Umfangsabschnitt der Verlauf der Stentfeder 12^{II}, die andere Begrenzung der Verlauf der Stentfeder 12^{III}.

Je nach Gefäß, in welches die intraluminale Gefäßprothese 10 implantiert werden soll, kann es notwendig sein, dass die Gefäßprothese 10 mehrere Fenestrierungs-Schnitte 16 aufweist. Insbesondere dann, wenn das Gefäß mehrere abgehende Gefäße aufweist. In einer bevorzugten Ausgestaltung weist die intraluminale Gefäßprothese 10 mindestens so viele Fenestrierungs-Schnitte 16 auf, wie abgehende Seitenäste in dem Bereich vorliegen, welcher durch die Gefäßprothese 10 überbrückt werden soll. Die abgehenden Seitenarme der Gefäße können erfindungsgemäß versorgt werden durch eine zweite Gefäßprothese, die durch die Fenestrierungs-Schnitte 16 gelegt wird, oder durch einfaches Öffnen der Klappen. Hierfür ist die Gefäßprothese 10 derart im Gefäß implantiert, dass der klappenartige Zugang zum abgehenden Seitenarm zeigt.

Fig. 2 zeigt eine zweite schematische Darstellung einer erfindungsgemäßen intraluminalen Gefäßprothese 10. Hierbei handelt es sich um eine vollständige Gefäßprothese 10 und nicht wie in Fig. 1 nur um ein Ausschnitt einer Gefäßprothese 10. Die gezeigte Gefäßprothese 10 weist vier Stentfedern 12 auf, welche zumindest teilweise an dem Prothesenmaterial 13 über eine Naht 14 befestigt sind. Die Stentfedern 12 weisen in dieser Darstellung unterschiedliche Amplituden auf. Die Form der Stentfedern 12 richtet sich insbesondere nach der Beschaffenheit des Gefäßes in welches die Gefäßprothese 10 implantiert werden soll. Besonders steife Gefäßprothesen 10 weisen bevorzugt sehr viele Stentfedern 12, die bevorzugt untereinander zu einer netzartigen Struktur miteinander verbunden sind, auf. Weniger steife Gefäßprothesen, die bspw. für dünnerwandige Gefäße eingesetzt werden können, weisen bevorzugt Stentfedern 12 mit größeren Amplituden auf. Ferner sind die einzelnen Stentfedern 12 in dieser Ausgestaltung beabstandet voneinander am Prothesenmaterial 13 angebracht.

Die intraluminale Gefäßprothese 10 weist in dieser Darstellung zwei Fenestrierungs-Schnitte 16 auf. Diese befinden sich in einem Bereich im Prothesenmaterial 13 der von zwei Stentfedern 12 eingegrenzt ist. Die Fenestrierungs-Schnitte 16 weisen an ihren Schnittenden jeweils ein Marker 17 auf. Dieser Marker 17 enthält oder besteht vollständig aus röntgendichtem Material, sodass bei der Implantierung der Gefäßprothese 10 die Position der Gefäßprothese 10 und insbesondere der Fenestrierungs-Schnitte 16 im Gefäß bestimmt werden kann.

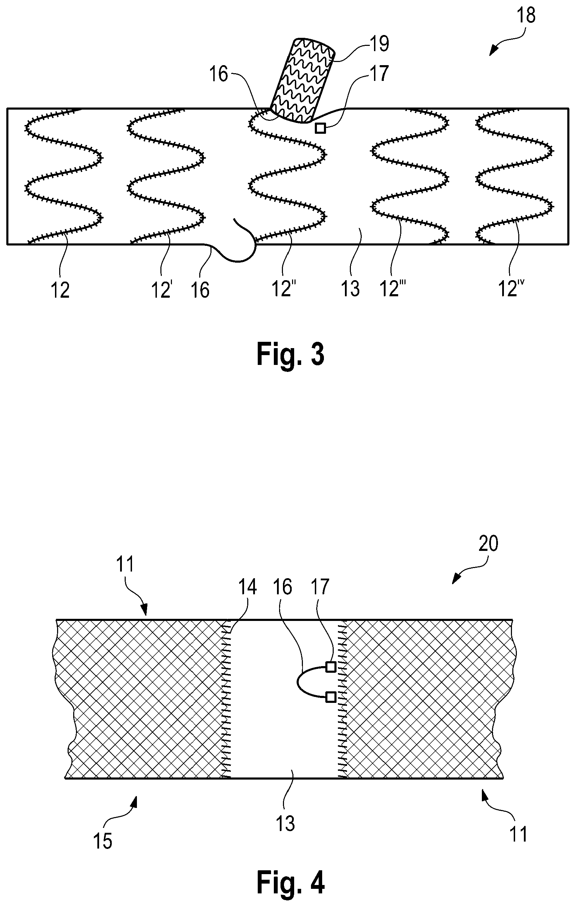

Fig. 3 zeigt eine schematische Darstellung eines Ausschnitts einer erfindungsgemäßen intraluminalen Gefäßprothese 18, mit eingeführtem hohlzylindrischem Seitenkörper 19. Die in Fig. 3 dargestellte Gefäßprothese 18 ähnelt im Wesentlichen der Gefäßprothese 10, gezeigt in Fig. 1. Die Gefäßprothese 18 weist zwei Fenestrierungs-Schnitte 16 auf, wobei der klappenartige Zugang einmal nach außen hin aufgeklappt vorliegt und einmal einen zusätzlichen, durch eine zweite Gefäßprothese 19 gebildeten Seitenkörper aufweist. Sowohl durch den aufgeklappten Zugang, als auch durch den Seitenkörper können abgehende Seitengefäße versorgt werden.

Der Seitenkörper, bzw. die zweite Gefäßprothese 19, ist in dieser Darstellung ausgestaltet als gecoverte Gefäßprothese. Andere hohlzylindrische Gefäßprothesen sind auch möglich, bspw. Gefäßprothesen ohne Prothesenmaterial, selbstexpandierbare Gefäßprothesen, ballonexpandierbare Gefäßprothesen etc.

Fig. 4 zeigt eine beispielhafte, nicht erfindungsgemäße schematische Darstellung eines Ausschnitts einer intraluminalen Gefäßprothese 20. Diese weist zwei Stent-Gerüste 11 auf, welche jeweils eine maschenartige bzw. netzartige Form aufweisen. Das Stent-Gerüst 11 ist über eine Naht 14 mit dem Prothesenmaterial 13 verbunden. Das Stent-Gerüst 11 ist in dieser Darstellung nicht ummantelt von Prothesenmaterial 13 wie es in den Figuren 1 bis 3 gezeigt ist. Das Stent-Gerüst 11 und das Prothesenmaterial 13 bilden zusammen einen hohlzylindrischen Grundkörper 15.

Die Gefäßprothese 20 weist im Prothesenmaterial 13 ein Fenestrierungs-Schnitt 16 auf, welcher an seinen Schnittenden jeweils ein Marker 17 aufweist.

Gemäß einer nicht dargestellten Ausgestaltung kann das Stent-Gerüst 11 der erfindungsgemäßen Gefäßprothese jede bekannte Form an Stent-Elementen 12 aufweisen. Die Stent-Elemente 12 können bspw. selbstexpandierbar oder ballonexpandierbar sein, aus Stentfedern, Stentringen, Stentnetzen und dergleichen ausgestaltet sein. Das Stent-Gerüst 11, bei dem die einzelnen Stent-Elemente 12 untereinander nicht verbunden sind, weist zur Ausbildung eines hohlzylindrischen Grundkörpers 15 Prothesenmaterial 13 auf. Das Prothesenmaterial 13 dient in diesem Fall als Verbindung zwischen den einzelnen Stent-Elementen 12. Demnach weist die erfindungsgemäße Gefäßprothese zumindest teilweise ein Prothesenmaterial 13 auf, welches einzelne Stent-Elemente 12 aufweist und mit diesen verbunden bzw. vernäht ist.

## Patentansprüche

1. Intraluminale Gefäßprothese (10, 18, 20) zur Implantation in ein Blutgefäß, mit einem Stent-Gerüst (11) und einem an dem Stent-Gerüst (11) befestigten Prothesenmaterial (13), wobei die Gefäßprothese (10, 18, 20) einen hohlzylindrischen Grundkörper (15) mit einem hierdurch führenden Lumen und einem umfänglich geschlossenem Mantel umfasst, **dadurch gekennzeichnet, dass**
im Prothesenmaterial (13) der Gefäßprothese (10, 18, 20) zumindest ein im Wesentlichen U- oder V-förmiger Fenestrierungs-Schnitt (16) vorgesehen ist, der derart dimensioniert und ausgebildet ist, dass über diesen zum Lumen der Gefäßprothese (10, 18, 20) ein klappenartiger Zugang für zumindest einen von dem hohlzylindrische Grundkörper (15) abgehenden Seitenast ausbildbar ist, und dass das Stent-Gerüst (11) der intraluminalen Gefäßprothese (10, 18, 20) aus in ihrer Längsrichtung hintereinander angeordneten, aber nicht miteinander verbundenen Ringen aus mäanderförmig umlaufenden Stützen besteht und der zumindest eine Fenestrierungs-Schnitt (16) in einem Umfangsabschnitt U zwischen zwei hintereinander angeordneten Ringen vorgesehen ist, und dass am ersten und am zweiten Ende der Gefäßprothese (10, 18, 20) jeweils ein Stentring vorgesehen ist, der mit dem Stent-Gerüst (11) verbunden ist.

2. Intraluminale Gefäßprothese (10, 18, 20) nach Anspruch 1, **dadurch gekennzeichnet, dass** die intraluminale Gefäßprothese (10, 18, 20) insgesamt zwischen einem und neun Fenestrierungs-Schnitten (16) auf dem Umfangsabschnitt U aufweist.

3. Intraluminale Gefäßprothese (10, 18, 20) nach einem der Ansprüche 1 oder 2, **dadurch gekennzeichnet, dass** sich das Stent-Gerüst (11) über die gesamte Länge der Gefäßprothese (10, 18, 20) erstreckt.

4. Intraluminale Gefäßprothese (10, 18, 20) nach einem der Ansprüche 1 oder 2, **dadurch gekennzeichnet, dass** sich das Stent-Gerüst (11) nicht über die gesamte Länge der Gefäßprothese (10, 18, 20) erstreckt, derart, dass zumindest ein ungestenteter Bereich ausgebildet ist.

5. Intraluminale Gefäßprothese (10, 18, 20) nach einem der Ansprüche 1 bis 4, **dadurch gekennzeichnet, dass** der zumindest eine Fenestrierungs-Schnitt (16) eine Schnittlänge von zwischen 2 mm bis 10 mm, vorzugsweise zwischen 5 mm und 7 mm aufweist.

6. Intraluminale Gefäßprothese (10, 18, 20) nach einem der Ansprüche 1 bis 5, **dadurch gekennzeichnet, dass** bei Vorliegen von mehreren Fenestrierungs-Schnitten (16) diese eine gleiche oder unterschiedliche Schnittlänge aufweisen.

7. Intraluminale Gefäßprothese (10, 18, 20) nach einem der Ansprüche 1 bis 6, **dadurch gekennzeichnet, dass** die intraluminale Gefäßprothese (10, 18, 20) neben dem hohlzylindrischen Grundkörper (15) ferner zumindest einen hohlzylindrischen Seitenkörper, der vorzugsweise durch eine zweite Gefäßprothese (19) gebildet ist, umfasst, welcher über den klappenartigen Zugang mit der Gefäßprothese (10, 18, 20) verbindbar und verbunden ist.

8. Intraluminale Gefäßprothese (10, 18, 20) nach einem der Ansprüche 1 bis 7, **dadurch gekennzeichnet, dass** sich auf der intraluminalen Gefäßprothese ein Marker (17) befindet, der ein röntgendichtes Material enthält oder vollständig aus röntgendichtem Material besteht, wobei der Marker (17) insbesondere an den Endpunkten des zumindest einen Fenestrierungs-Schnitts (16) und/oder entlang des Fenestrierungs-Schnitts (16) vorliegen.

9. Intraluminale Gefäßprothese (10, 18, 20) nach einem der Ansprüche 1 bis 8, **dadurch gekennzeichnet, dass** der klappenartige Zugang für zumindest einen nach Innen ins Lumen der Gefäßprothese (10, 18, 20) und/oder nach Außen abgehenden Seitenast ausbildbar ist.

10. Verfahren zur Herstellung einer intraluminalen Gefäßprothese (10, 18, 20) zur Implantation in ein Blutgefäß nach einem der Ansprüche 1 bis 9, **dadurch gekennzeichnet, dass** der Fenestrierungs-Schnitt (16) im Prothesenmaterial (13) mittels einer thermischen Behandlung des Prothesenmaterials (13) erfolgt.

11. Verfahren zur Herstellung einer intraluminalen Gefäßprothese (10, 18, 20) zur Implantation in ein Blutgefäß nach Anspruch 10, **dadurch gekennzeichnet, dass** der Fenestrierungs-Schnitt (16) mittels eines Laserwerkzeugs oder eines Werkzeugs eingebracht wird.

## Claims

1. An intraluminal vascular prosthesis (10, 18, 20) for implantation into a blood vessel, with a stent framework (11) and a prosthesis material (13) secured onto the stent framework (11), wherein the vascular prosthesis (10, 18, 20) has a hollow-cylindrical body (15) with a lumen passing there through and a circumferentially closed jacket, **characterized in that**
at least one substantially U-shaped or V-shaped fenestration-cut (16) in the prosthesis material (13) of the vascular prosthesis (10, 18, 20) is provided, which is dimensioned and formed, such, that via the fenestration-cut a flap-like access to the lumen of the vascular prosthesis (10, 18, 20) for at least one side branch branching off from the hollow-cylindrical body (15) can be formed, and that the stent framework (11) of the intraluminal vascular prosthesis (10, 18, 20) is made of rings of meandering circumferential struts arranged one behind the other in its longitudinal direction, but not connected to one another, and that the at least one fenestration-cut (16) is provided in at least one circumferential portion U between two successively arranged rings, and that a respective stent ring at the first and second end of the vascular prosthesis (10, 18, 20) is provided, which is connected to the stent framework (11).

2. The intraluminal vascular prosthesis (10, 18, 20) according to claim 1, **characterized in that** the intraluminal vascular prosthesis (10, 18, 20) has between one and nine fenestration-cuts (16) on at least one circumferential portion U.

3. The intraluminal vascular prosthesis (10, 18, 20) according to any one of claims 1 or 2, **characterised in that** the stent framework (11) extends over the entire length of the vascular prosthesis (10, 18, 20).

4. The intraluminal vascular prosthesis (10, 18, 20) according to any one of claims 1 or 2, **characterised in that** the stent framework (11) does not extend over the entire length of the vascular prosthesis (10, 18, 20) such that at least one un-stented region is formed.

5. The intraluminal vascular prosthesis (10, 18, 20) according to any one of claims 1 to 4, **characterised in that** the at least one fenestration-cut (16) has a cut length of between 2 mm to 10 mm, preferably between 5 mm and 7 mm.

6. The intraluminal vascular prosthesis (10, 18, 20) according to any one of claims 1 to 5, **characterised in that** in case of a plurality of fenestration-cuts (16) they have the same or different length of the cut.

7. The intraluminal vascular prosthesis (10, 18, 20) according to any one of claims 1 to 6, **characterised in that** the intraluminal vascular prosthesis (10, 18, 20) further has, in addition to the hollow-cylindrical body (15), at least one hollow-cylindrical side body, which is preferably formed by a second vascular prosthesis (19) and which is connectable to the vascular prosthesis (10, 18, 20) via the flap-like access.

8. The intraluminal vascular prosthesis (10, 18, 20) according to any one of claims 1 to 7, **characterised in that** a marker (17) is located on the intraluminal vascular prosthesis, which marker contains a radiopaque material or is made entirely of radiopaque material, wherein the marker (17) is provided in particular at the end points of the at least one fenestration-cut (16) and/or along the fenestration-cut (16).

9. The intraluminal vascular prosthesis (10, 18, 20) according to any one of claims 1 to 8, **characterised in that** the flap-like access is formable for at least one side branch extending inwards into the lumen of the vascular prosthesis (10, 18, 20) and/or outwards.

10. Method for producing an intraluminal vascular prosthesis (10, 18, 20) according to any one of claims 1 to 9, **characterized in that** the fenestration-cut (16) in the prosthesis material (13) is made by means of a thermal treatment of the prosthesis material (13).

11. Method for producing an intraluminal vascular prosthesis (10, 18, 20) according to claim 10, **characterized in that** the fenestration-cut (16) is made by means of a laser instrument or of a tool.

## Revendications

1. Prothèse vasculaire (10, 18, 20) intraluminale destinée à être implantée dans un vaisseau sanguin, comportant une armature d'endoprothèse (11) et un matériau prothétique (13) fixé à l'armature d'endoprothèse (11), dans laquelle la prothèse vasculaire (10, 18, 20) comprend un corps de base (15) cylindrique creux comportant une lumière le traversant et une enveloppe fermée sur sa périphérie, **caractérisée en ce que**
dans le matériau prothétique (13) de la prothèse vasculaire (10, 18, 20) est prévue au moins une incision de fenestration (16) sensiblement en forme de U ou de V qui est dimensionnée et réalisée de telle sorte que, par l'intermédiaire de celle-ci, un accès en forme de clapet vers la lumière de la prothèse vasculaire (10, 18, 20) peut être formé pour au moins une branche latérale partant du corps de base (15) cylindrique creux, **et en ce que** l'armature d'endoprothèse (11) de la prothèse vasculaire (10, 18, 20) intraluminale est constituée d'anneaux disposés les uns derrière les autres dans leur direction longitudinale, mais non reliés les uns aux autres, et constitués de supports s'étendant en forme de méandres, et l'au moins une incision de fenestration (16) est prévue dans une section périphérique U entre deux anneaux disposés l'un derrière l'autre, **et en ce qu'**est prévu à la première et à la seconde extrémité de la prothèse vasculaire (10, 18, 20) respectivement un anneau d'endoprothèse qui est relié à l'armature d'endoprothèse (11).

2. Prothèse vasculaire (10, 18, 20) intraluminale selon la revendication 1, **caractérisée en ce que** la prothèse vasculaire (10, 18, 20) intraluminale présente au total entre une et neuf incisions de fenestration (16) sur la section périphérique U.

3. Prothèse vasculaire (10, 18, 20) intraluminale selon l'une des revendications 1 ou 2, **caractérisée en ce que** l'armature d'endoprothèse (11) s'étend sur toute la longueur de la prothèse vasculaire (10, 18, 20).

4. Prothèse vasculaire (10, 18, 20) intraluminale selon l'une des revendications 1 ou 2, **caractérisée en ce que** l'armature d'endoprothèse (11) ne s'étend pas sur toute la longueur de la prothèse vasculaire (10, 18, 20), de telle sorte qu'au moins une zone n'appartenant pas à l'endoprothèse est réalisée.

5. Prothèse vasculaire (10, 18, 20) intraluminale selon l'une des revendications 1 à 4, **caractérisée en ce que** l'au moins une incision de fenestration (16) présente une longueur d'incision allant d'entre 2 mm à 10 mm, de préférence entre 5 mm et 7 mm

6. Prothèse vasculaire (10, 18, 20) intraluminale selon l'une des revendications 1 à 5, **caractérisée en ce que,** en présence de plusieurs incisions de fenestration (16), celles-ci présentent des longueurs d'incisions identiques ou différentes.

7. Prothèse vasculaire (10, 18, 20) intraluminale selon l'une des revendications 1 à 6, **caractérisée en ce que** la prothèse vasculaire (10, 18, 20) intraluminale comprend en outre, outre le corps de base (15) cylindrique creux, au moins un corps latéral cylindrique creux qui est de préférence formé par une seconde prothèse vasculaire (19), lequel peut être relié et est relié à la prothèse vasculaire (10, 18, 20) par l'intermédiaire de l'accès en forme de clapet.

8. Prothèse vasculaire (10, 18, 20) intraluminale selon l'une des revendications 1 à 7, **caractérisée en ce que** sur la prothèse vasculaire intraluminale se trouve un marqueur (17) qui contient un matériau radio-opaque ou qui est entièrement constitué d'un matériau radio-opaque, dans laquelle le marqueur (17) est en particulier présent aux points d'extrémité de l'au moins une incision de fenestration (16) et/ou le long de l'incision de fenestration (16).

9. Prothèse vasculaire (10, 18, 20) intraluminale selon l'une des revendications 1 à 8, **caractérisée en ce que** l'accès en forme de clapet peut être réalisé pour au moins une branche latérale s'étendant vers l'intérieur dans la lumière de la prothèse vasculaire (10, 18, 20) et/ou vers l'extérieur.

10. Procédé pour la fabrication d'une prothèse vasculaire (10, 18, 20) intraluminale destinée à être implantée dans un vaisseau sanguin selon l'une des revendications 1 à 9, **caractérisé en ce que** l'incision de fenestration (16) dans le matériau prothétique (13) est effectuée au moyen d'un traitement thermique du matériau prothétique (13).

11. Procédé pour la fabrication d'une prothèse vasculaire (10, 18, 20) intraluminale destinée à être implantée dans un vaisseau sanguin selon la revendication 10, **caractérisé en ce que** l'incision de fenestration (16) est introduite au moyen d'un outil laser ou d'un outil.
